# EUROPÄISCHE PATENTANMELDUNG

(11) **EP 4 023 637 A1**
(43) Veröffentlichungstag der Anmeldung: **06.07.2022**
(21) Anmeldenummer: 20217816.6
(22) Anmeldetag: 30.12.2020
(51) Int. Cl.: C07C 319/20, C07C 323/60, C07D 241/08, A23K 20/142, A23K 50/80

(54) **VERFAHREN ZUR DIASTEREOMERENREINEN HERSTELLUNG VON DL/LD-METHIONYL-METHIONIN**

(71) Anmelder: Evonik Operations GmbH, 45128 Essen (DE); Evonik Antwerpen NV, 2040 Antwerpen (BE)
(72) Erfinder: BRAUNE, Sascha, 2180 Antwerpen (BE); VAN EESTER, Geert, 2100 Antwerpen (BE); HASSELBACH, Hans-Joachim, 63571 Geinhausen (DE); DYBALLA, Claudia, 63517 Rodenbach (DE)
(74) Vertreter: Evonik Patent Association

(57) **Zusammenfassung**

Die Erfindung betrifft insbesondere ein Verfahren zur Herstellung von DL/LD-Methionin-diketopiperazin (I) 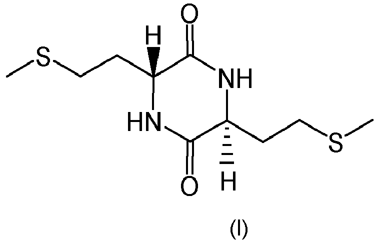 dadurch gekennzeichnet, dass man eine DD/LL-Methionyl-methionin enthaltende wässrige Lösung oder Suspension, die einen pH-Wert von 3 bis 9, gemessen mit einer pH-Elektrode bei 50 °C aufweist, solange bei einer Temperatur von 150 bis 220°C, vorzugsweise bei 170 bis 210 °C umsetzt, bis eine wässrige Lösung oder Suspension enthaltend mindestens einen Anteil von 30 Mol %, vorzugsweise 50 Mol %, insbesondere 60 Mol% DL/LD-Methionindiketopiperazin bezogen auf den Gesamtgehalt an DL/LD- und DD/LL-Methionindiketopiperazin im Reaktionsgemisch entstanden ist.

Dieses Verfahren ist Teil eines Gesamtverfahrens zur Herstellung von DL/LD-Methionyl-methionin, welches aus dem primär erzeugten DL/LD-Methionin-diketopiperazin (I) durch Hydrolyse erhalten wird.

## Beschreibung

### Einleitung

Die vorliegende Erfindung betrifft ein Verfahren zur diastereomerenreinen Herstellung von DL/LD-Methionyl-methionin aus DD/LL-Methionyl-methionin mittels Cyclisierung und Epimerisierung zum DL/LD- 2,6-Bis(methionyl)-1,4-diketopiperazin (I, meso-Methionin-DKP, meso-*met*-DKP) und anschließende Hydrolyse. Unter "diastereomerenrein" oder auch "im wesentlichen diastereomerenrein" wird in diesem Zusammenhang ein Produkt verstanden, das zumindest 90 Mol% des gewünschten DL/LD-Diastereomeren enthält, vorzugsweise mindestens 95 bis 97 Mol% am Gesamtgehalt an enthaltenem Methionyl-methionin (Met-Met).

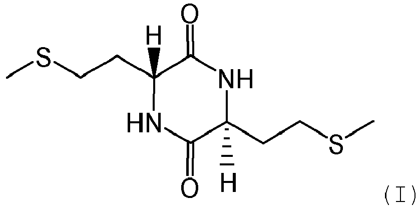

WO2010043558 A1 offenbart ein Verfahren zur Herstellung von Methionyl-methionin (Met-Met), dem homologen Dipeptid aus DL-Methionin, welches vor allem als hochwertige Methioninquelle bei der Fütterung von in Aquakulturen gehaltenen Fischen und Krustentieren zunehmend Anwendung findet.

Aufgrund seiner zwei Stereozentren (zwei asymmetrische C-Atome) liegt das Produkt in Form von zwei Diastereomeren vor, dem DL,LD-Met-Met und dem DD,LL-Met-Met, also in Form von zwei konfigurationsisomeren Enantiomerenpaaren, die zwar beide von den Tieren gut verwertet werden können, die aber deutlich unterschiedliche physikalische Eigenschaften aufweisen, was in der praktischen Handhabung zu gewissen Problemen führen kann:
Es hat sich herausgestellt, dass die beiden Diastereomeren unterschiedliche Kristallstrukturen bilden. Während DLLD-Met-Met ein kugelförmiges Agglomerat in der Endproduktfällung bildet, kristallisiert DDLL-Met-Met in Nadeln aus. Damit sind unterschiedliche Rieselfähigkeit und Verklumpungsneigung sowie unterschiedliche Löslichkeit verbunden.

Während DLLD-Met-Met bei Raumtemperatur eine Wasserlöslichkeit von nur 0,4 g/l aufweist, liegt die Wasserlöslichkeit von DDLL-Met-Met mit 21,0 g/l viel höher. Eine hohe Wasserlöslichkeit ist aber für die Anwendung in Aquakulturen eher ungünstig, da es dadurch via Leaching aus dem Futterpellet zu Wertstoffverlusten kommen kann. Das Produkt aus dem Herstellungsprozess gemäß WO2010043558 A1 weist typischerweise ein Verhältnis von DLLD- / DDLL-Met-Met von 60 /40 auf. Ein Produkt mit deutlich höherem Anteil an DLLD-Met-Met wäre daher wünschenswert. Im übrigen zeigt das bisher bekannte Produkt relativ niedrige Schüttgewichte von max. 500 kg/m³, nur mäßige bis relative schlechte Rieselfähigkeiten von 3 (befriedigend) bis 5 (mangelhaft) und relativ niedrige Mindestzündenergie von MIE < 3 mJ, ist also vergleichsweise leicht entzündlich, so dass auch diese Eigenschaften verbesserungswürdig erschienen sind.

Die Synthese des 2,5-Diketopiperazins ausgehend vom Methioninmethylesterwird bereits in der Offenlegungsschrift DE 2 261 926 aus dem Jahre 1972 offenbart. Darin wird offenbart, dass durch Erhitzen des Isopropylesters von Methionin 3,6-Bis[2-(methylthio)ethyl]-2,5-piperazindion (Methionindiketopiperazin, DKP) gebildet wird. Auch die Veröffentlichung von Baker, D.H. et al. (Journal of Nutrition, Band 114, Nr. 2, 1984, Seite 292-297) bezieht sich auf dieses Verfahren zur Herstellung von Diketopiperazin. Allerdings ist die Verwendung von Methioninisopropylester als Ausgangsmaterial zu teuer und daher unwirtschaftlich.

### Aufgabe

Es war daher Aufgabe der zugrundeliegenden Erfindung, ein Verfahren zur Herstellung einer DLLD, DDLL-Met-Met- Qualität, bereitzustellen, die einen deutlich höheren Anteil von DLLD-Met-Met an der Gesamtmenge der Diastereomeren im Vergleich zum Produkt gemäß des Stands der Technik aufweist sowie die Bereitstellung eines Produktes, das zudem möglichst bessere Handhabungs- und sicherheitstechnische Eigenschaften aufweisen sollte im Vergleich zum bisherigen Produkt.

### Beschreibung der Erfindung

Die genannte Aufgabe wird mit der vorliegenden Erfindung gelöst, indem praktisch nur noch das weniger lösliche Enantiomerenpaar, das DLLD-Met-Met, isoliert wird und das unerwünschte DDLL-Met-Met wieder in das unmittelbare Vorprodukt DKP zurückverwandelt wird und dabei simultan epimerisiert wird.
Einen grundlegenden Anteil an der Lösung der oben genannten Aufgaben liefert erfindungsgemäß somit ein
Verfahren zur Herstellung von DL/LD-Methionindiketopiperazin (I) 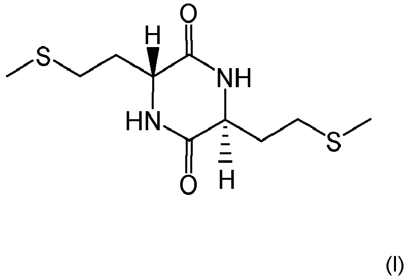 dadurch gekennzeichnet, dass man eine DD/LL-Methionyl-methionin enthaltende wässrige Lösung oder Suspension, die einen pH-Wert von 3 bis 9, vorzugsweise 4 bis 6, insbesondere von ca.5, gemessen mit einer pH-Elektrode bei 50 °C aufweist, solange bei einer Temperatur von 150 bis 220°C, vorzugsweise von 170 bis 210°C umsetzt, bis eine wässrige Lösung oder Suspension enthaltend mindestens einen Anteil von 30 Mol %, vorzugsweise 50 Mol %, insbesondere 60 Mol% DL/LD-Methionindiketopiperazin bezogen auf den Gesamtgehalt an DL/LD- und DD/LL-Methionindiketopiperazin im Reaktionsgemisch entstanden ist (siehe Beispiele 3 bis 9). Dieses kann anschließend zum entsprechenden Methionylmethionin-Isomerengemisch hydrolysiert werden. Höhere pH-Werte von > 9 oder niedrigere pH-Werte von < 3 führen zu niedrigeren Ausbeuten bzw. ungünstigeren Endverhältnissen von DLLD/DDLL-DKP, wie die Vergleichsbeispiele 1 und 2 zeigen.

In den durchgeführten Untersuchungen wurde beispielsweise eine natriumsulfathaltige, nahezu ausschließlich DDLL-Met-Met enthaltende Mutterlauge aus dem technischen Met-Met-Prozess gemäß WO2010043558 A1 auf Temperaturen über 170°C erhitzt und vorzugsweise etwa eine Stunde gehalten. Dabei bildete sich bei pH 5 nicht nur quantitativ DKP, sondern es wurde überraschenderweise gleichzeitig eine Epimerisierung des zu erwartenden DDLL- DKPs (> 95 %) zu einem Gemisch aus DLLD-DKP / DDLL-DKP mit einem Verhältnis von etwa 60 /40 beobachtet. Diese überraschende Epimerisierung eines Stereozentrums des DKPs ist die Grundlage dafür, dass nach einer fest / flüssig- Trennung das auf diesem Wege erhaltene DKP in den bestehenden Prozess zur Umsetzung von DKP zu Met-Met zurückgespeist werden kann. Parallel wird dadurch ein natriumsulfathaltiger Abfallstrom generiert, der eine deutlich verminderte Belastung an TOC/ COD (Verhältnis aus Total Organic Carbon und Chemical Oxygen Demand) als bisher aufweist. Die Verminderung der TOC/ COD- Belastung des Abwasserstroms resultiert aus der deutlich geringeren Löslichkeit von DKP gegenüber Met-Met.

Ein solches Verfahren wird vorzugsweise so durchgeführt, dass die Gesamtkonzentration an DL/LD- und DD/LL-Methionyl-methioninäquivalenten in Form von DL/LD- und DD/LL-Methionindiketopiperazin im Reaktionsgemisch am Ende der Reaktion im Bereich von 0,1 bis 18 Gew.%, besonders bevorzugt im Bereich von 1 bis 15 Gew.%, liegt. Am einfachsten und vorteilhaftesten ist es aber, bei der im Verfahren sich einstellenden Konzentration von ca. 3 Gew% zu arbeiten, weil das ohne zusätzlichen Energieaufwand zur Aufkonzentrierung erreicht werden kann und trotzdem ausreichend viel DKP (und somit TOC) aus dem Abfallstrom isoliert werden kann.

Dabei wird die Umsetzung vorzugsweise bei einer Temperatur von 175 bis 210 °C durchgeführt. Das hat wiederum den Vorteil, dass in technisch sinnvoll realisierbaren Reaktionszeiten die Umsetzung erfolgt. Eine darüber hinausgehende Erhöhung der Temperatur ist jedoch durch eine parallel einsetzende Zersetzung an der Methylthiogruppe limitiert.

Außerdem kann im erfindungsgemäßen Verfahren eine DD/LL-Methionyl-methionin enthaltende wässrige Lösung oder Suspension, die bereits DL/LD-Methionyl-methionin enthält eingesetzt werden, nämlich in etwa bis zu einem Anteil von 30 (Mol% DL/LD-Methionylmethionin bezogen auf ihren Gesamtgehalt an DD/LL- und DL/LD -Methionyl-methionin. Dies ist vorteilhaft, weil je nach den Verfahrensbedingungen auch Chargen anfallen können, die etwas höhere Anteile an DL/LD-Methionyl-methionin enthalten und die so problemlos verarbeitet werden können.

Im Gegensatz zur vorliegenden Erfindung offenbart WO 2010/043558 A1 eine alternative Ausführungsform des dort beschriebenen erfindungsgemäßen Verfahrens umfassend die folgenden Schritte:
i) Umsetzung eines Harnstoffderivates der allgemeinen Formel II, 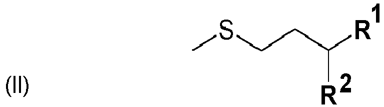 wobei die Reste R1 und R2 in den Harnstoffderivaten IIa, IIb, IIc, IId, IIe, IIf und IIg wie folgt definiert sind:
   wobei
   IIa: R¹ = COOH, R² = NHCONH₂
   IIb: R¹ = CONH₂, R² = NHCONH₂
   IIc: R¹ = CONH₂, R² = NH₂
   IId: R¹-R² = -CONHCONH-
   IIe: R¹ = CN, R²=OH
   IIf: R¹ = CN, R²=NH₂
   IIg: R¹ = = O, R² = H
   bedeutet,
   zu einem Diketopiperazin der Formel III 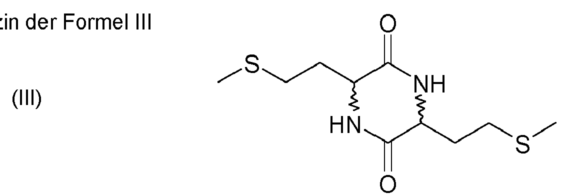
ii) Umsetzung des Diketopiperazins zum DL-Methionyl-DL-methionin. Dieses Verfahren umfasst die in Schema 3 der WO2010043558 A1 aufgezeigten Methoden A, B, C und D. Bei diesen Verfahren wird Diketopiperazin (III) als Zwischenprodukt gebildet.
Dabei ist bevorzugt, dass die Umsetzung des Harnstoffderivates zum Diketopiperazin bei einer Temperatur von 50°C bis 200°C, vorzugsweise von 100°C bis 180°C und besonders bevorzugt von 140°C bis 170°C durchgeführt wird.
In einem bevorzugten Verfahren erfolgt die Umsetzung des Harnstoffderivates zum Diketopiperazin unter Druck, bevorzugt bei einem Druck von 3 bis 20 bar, besonders bevorzugt bei einem Druck von 6 bis 15 bar.
Vorzugsweise erfolgt die Umsetzung des Harnstoffderivates zum Diketopiperazin in Gegenwart einer Base. Dabei ist die Base bevorzugt ausgewählt aus der Gruppe Stickstoff-haltiger Basen, NH₄HCO₃, (NH₄)₂CO₃, KHCO₃, K₂CO₃, NH₄OH/CO₂-Mischung, Carbamatsalze, Alkali- und Erdalkalibasen .
In einem weiteren bevorzugten Verfahren erfolgt die Umsetzung des Harnstoffderivates zum Diketopiperazin durch Reaktion mit Methionin. Dabei ist ein Verhältnis von Harnstoffderivat zu Methionin von 1:100 bis 1:0,5 bevorzugt.
In einem weiteren bevorzugten Verfahren erfolgt die Umsetzung des Diketopiperazin zum DL-Methionyl-DL-methionin durch saure Hydrolyse.

Im Gegensatz zum vorstehend beschriebenen Verfahren gemäß WO 2010/043558 A1 zielt die vorliegende Erfindung nicht darauf ab, das weniger erwünschte DD,LL-Met-Met-Diastereomer direkt in das erwünschte DL,LD-Met-Met-Diastereomer zu überführen, sondern der entscheidende erfindungsgemäße Schritt der simultanen Cyclisierung und Epimerisierung ausgehend vom DD,LL-Met-Met-Diastereomer zum DLLD-DKP-Gemisch gelingt dabei überraschenderweise durch die Anwendung der oben genannten Reaktionsbedingungen. Das DLLD-DKP- Gemisch kann dann anschließend in hohen Ausbeuten zum entsprechenden DLLD-Met-Met-Gemisch hydrolysiert werden. Die Ausbeuten sind dabei deutlich höher als beim vorstehend genannten Verfahren gemäß WO 2010/043558 A1 und praktisch ohne Entstehung von Met als Nebenprodukt.

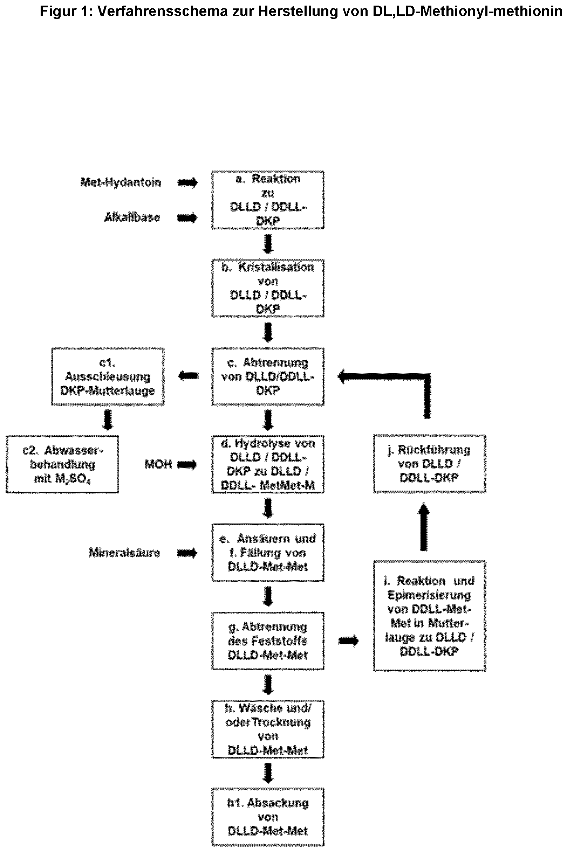

Ein weiterer Gegenstand der vorliegenden Erfindung ist das folgende Gesamtverfahren (vgl. Figur 1) zur Gewinnung von diastereomerenreinem DL/LD-Methionylmethionin, dadurch gekennzeichnet, dass man
a. Methionin-Hydantoin zusammen mit Alkalibase umsetzt zu einem Diastereomerengemisch aus DL/LD-Methionindiketopiperazin und DD/LL-Methionindiketopiperazin (DLLD-DKP und DDLL-DKP)
b. das DLLD-DKP und DDLL-DKP aus a. durch Einengen oder Abkühlen der Reaktionslösung aus a. zur Kristallisation bringt
c. das auskristallisierte DLLD-DKP und DDLL-DKP abtrennt von der DKP-Mutterlauge
d. das abgetrennte DLLD-DKP und DDLL-DKP in Wasser einmischt und das Gemisch durch Zugabe entsprechender Mengen MOH (M=Alkali) auf einen pH-Wert von 10 bis 14, vorzugsweise 13, gemessen mit einer pH-Elektrode bei 20 °C, bringt und bei einer Temperatur von 90 bis 150 °C und einer Verweilzeit von 30 bis 180 min zu einer entsprechenden wässrigen Lösung oder Suspension eines Diastereomerengemisches aus DL/LD-Methionylmethionin- und DD/LL-Methionylmethionin-Alkalisalz hydrolysiert,
e. den pH-Wert der Lösung oder Suspension aus d., gemessen bei 20°C, durch Zugabe entsprechender Mengen Säure auf < 9,0, vorzugsweise auf 4,6 bis 5,6, besonders bevorzugt auf 5,0 bis 5,2 absenkt (Ansäuern),
f. durch Verdampfen von Wasser und/ oder Abkühlen eine Suspension erzeugt (Fällung), die aus einem Feststoffanteil, der vorwiegend DL/LD-Methionylmethionin enthält, und Mutterlauge, die vorwiegend DD/LL-Methionylmethionin enthält, besteht, man
g. den Feststoffanteil aus f. von der Mutterlauge abtrennt, und man
h. den abgetrennten Feststoffanteil aus g. wahlweise wäscht und/oder trocknet, und dabei diastereomerenreines DL/LD-Methionylmethionin erhält und man
i. die verbleibende DD/LL-Methionylmethionin enthaltende Mutterlauge aus g. gemäß der oben beschriebenen Verfahrensweise (vgl. auch Anspruch 1) umsetzt, so dass eine wässrige Lösung oder Suspension enthaltend ein Diastereomerengemisch aus DL/LD-Methionindiketopiperazin und DD/LL-Methionindiketopiperazin entsteht mit einem Anteil von bis zu 60 Mol% DL/LD-Methionindiketopiperazin am Gesamtgehalt der DL/LD- und DD/LL-Methionylmethioninäquivalente im Reaktionsgemisch und
j. diese dann in Schritt b. zurückführt.

Die Trennung der diastereomeren Enantiomerenpaare kann durch fraktionierte Kristallisation bei Temperaturen über 50 °C und Met-Met-Konzentrationen unter 15%, bevorzugt unter 10% erzielt werden. Bei diesen Bedingungen lässt sich gezielt nahezu ausschließlich das Enantiomerenpaar aus DLLD-Met-Met fällen. Das DDLL-Met-Met verbleibt mit über 95% in Lösung. Eine solche Trennung ist mit dem Ausgangsprodukt, dem cyclischen Dipeptid DKP, das ein Verhältnis von DLLD-DKP / DDLL-DKP von 55 / 45 aufweist, nicht möglich

Dabei werden in Schritt d. vorzugsweise 0,9 bis 1,5 Moleq, besonders bevorzugt 1,0 bis 1,2 Moleq Base bezogen auf den Gesamtgehalt an DL/LD- und DD/LL-Methionyl-methioninäquivalenten im Reaktionsgemisch eingesetzt. Diese Verfahrensweise bewirkt eine praktisch quantitative Hydrolyse der DKP-Vorstufe zum Dipeptid.

Als Base in Schritt d. werden vorzugsweise Alkalimetallhydroxide verwendet, besonders bevorzugt NaOH oder KOH, weil diese eine ausreichend hohe Alkalität und Löslichkeit aufweisen, es sich dabei um relativ preiswerte aber gleichzeitig in hoher Qualität verfügbare Industriechemikalien handelt, die darüber hinaus im Hinblick auf eventuell im Endprodukt verbleibende Spuren völlig unproblematisch sind,

Als Säure in Schritt e. werden vorzugsweise starke Mineralsäuren wie z.B. Phosphorsäure, Salzsäure oder Schwefelsäure, insbesondere Schwefelsäure verwendet. Schwefelsäure bildet bei der Neutralisation mit NaOH oder KOH die entsprechenden Natrium- bzw. Kaliumsulfate, welche sich in der Abtrennung des Produkts DL/LD-Methionylmethionin als besonders hilfreich erwiesen haben, da besonders gut filtrierbare Produktkristalle insbesondere bei vorheriger Verwendung von NaOH gebildet werden.

In Schritt f. wird vorzugsweise bis zu einer Temperatur von > 50 °C abgekühlt. Das hat den Effekt, dass höchstens kleine Anteile an noch vorhandenem DDLL- Met-Met mit ausfallen, so dass im Produkt nur noch ein Anteil von weniger als 5 % des unerwünschten Diastereomeren DDLL- Met-Met verbleibt.

Der Prozess wird vorzugsweise so gefahren, dass die in Schritt f. erzeugte Suspension mindestens 1 Gew%, jedoch nicht mehr als 15 Gew.% Gesamtgehalt an DD/LL- und DL/LD -Methioninyl-methionin enthält, Auf diese Weise wird erreicht, dass relativ gut filtrierbare Kristalle, hauptsächlich bestehend aus. DLLD-Met-Met erzeugt werden können.

Die Trennung der Enantiomerenpaare kann also vorteilhaft durch fraktionierte Kristallisation bei Temperaturen über 50 °C und bei Met-Met-Konzentrationen unter 10% erzielt werden. Bei diesen Bedingungen lässt sich gezielt nahezu ausschließlich das Enantiomerenpaar aus DLLD-Met-Met fällen. Das DDLL-Met-Met verbleibt mit über 95% in Lösung. Eine solche Trennung ist mit dem Ausgangsprodukt, dem cyclischen Dipeptid DKP, das ein Verhältnis von DLLD-DKP / DDLL-DKP von ca. 55 / 45 aufweist, nicht möglich wie bereits ausgeführt.

Der in Schritt g. abgetrennten Feststoffanteil wird weiterreinigt und/oder getrocknet, wodurch ein pulverförmiges weitgehend diastereomerenreines DL/LD-Methionylmethionin erhalten wird, welches direkt geeignet ist zur Verwendung als Futtermittelzusatzstoff für Aquakulturen.

Ein weiterer Gegenstand der Erfindung ist somit ein
pulverförmiges weitgehend diastereomerenreines DL/LD-Methionylmethionin, erhältlich nach einem der oben beschriebenen erfindungsgemäßen Verfahren, welches geeignet ist zur Verwendung als Futtermittelzusatzstoff für Aquakulturen.

Ein solches pulverförmiges, weitgehend diastereomerenreines DL/LD-Methionylmethionin enthaltendes Produkt, weist die folgenden Merkmale auf:
a. Mindestens 97 Gew.% Gesamtgehalt an DD/LL- und DL/LD -Methionyl-methionin,
b. Mindestens 90 Mol% DL/LD-Methionylmethionin und maximal 10 Mol% DD/LL-Methionyl-methionin, vorzugsweise mindestens 95 Mol% an DL/LD-Methionyl-methionin und maximal 5 Mol% DD/LL-Methionyl-methionin, jeweils bezogen auf den Gesamtgehalt an DD/LL- und DL/LD - Methionyl-methionin,
c. Max. 1,0 Gew.% Restfeuchte,
d. Max. 2,0 Gew.% Sulfat(Asche) und
e. einer Korngrößenverteilung mit einem Median D50 von 80 bis 300 µm, vorzugsweise 150 bis 250 µm.

Das Produkt eignet sich insbesondere zur Verwendung als Futtermittelzusatzstoff für Aquakulturen, weil es im Wasser relativ schwerlöslich ist und damit kaum durch Auswaschen aus einem Futterpellet, in den es vorher eingearbeitet worden ist, verloren gehen kann.

Entsprechend ist ein weiterer Gegenstand der Erfindung die entsprechende Verwendung von weitgehend diastereomerenreinem DL/LD-Methionyl-methionin als Futtermittelzusatzstoff für Aquakulturen.
Nach einer fest / flüssig- Trennung und Trocknung erhält man hochreines AQUAVI^{®}Met- Met mit einem Diastereomerenverhältnis von ≥ 95 / ≤ 5 DLLD / DDLL-Met-Met. Dieses neue Produkt weist Schüttdichten von deutlich über 600 kg / m³ auf im Vergleich zum bisherigen DLLD / DDLL-Met-Met- Produktgemisch, dem Standardprodukt aus WO2010043558 A1, mit nur unter 500 kg / m³. Das neue Produkt zeigt auch ein deutlich verbessertes Fließverhalten, in der Regel von Rieselnote 3 oder besser (befriedigend bis gut) auf der Skala von 1 bis 6 gemessen mit Standardauslaufzylindern (vgl. Beispiele Analytische Methoden) im Vergleich zum bisherigen DLLD / DDLL-Met-Met- Produktgemisch, das in der Regel eine Rieselnote von 5 bis 6 aufweist. Darüber hinaus liegt die Mindestzündenergie des erfindungsgemäßen Produkts bei Raumtemperatur über 10 mJ und selbst bei 100°C noch über 3 mJ und ist somit nicht mehr als besonders zündempfindlich zu klassifizieren. Das ist von großem Vorteil für die sichere Handhabung des Produktes bzw. den dafür zu betreibenden Aufwand.

### Figur 1 zeigt das Schema zur Herstellung DL/LD-Methionyl-methionins

Das Schema umfasst die folgenden Schritte:
a. Reaktion von Methioninhydantoin mit Alkalibase zu DLLD/DDLL-Met-DKP
b. Kristallisation von DLLD/DDLL-Met-DKP
c. Abtrennung von DLLD/DDLL-Met-DKP
   c1. Ausschleusung von DKP-Mutterlauge
   c2. Abwasserbehandlung
d. alkalische Hydrolyse von DLLD/DDLL-Met-DKP zu DLLD/DDLL-Met-Met
e. Ansäuern und
f. Fällung von DLLD-Met-Met
g. Abtrennung des Feststoffs DLLD -Met-Met
h. Wäsche und/oder Trocknung von DLLD-Met-Met
   h1. Absackung von DLLD-Met-Met
i. Reaktion und Epimerisierung von DDLL -Met in DDLL -Met-haltiger Mutterlauge zu DLLD/DDLL-Met-DKP
j. Rückführung von DLLD/DDLL-Met-DKP in Schritt c.

### Beispiele

Die Beispiele wurden, wenn nicht anders angegeben, wie in der allgemeinen Verfahrensbeschreibung und gemäß der jeweils zugehörigen Angaben in Tabelle 1 durchgeführt.

### Analytische Methoden

### Bestimmung der Schüttdichte

Die Schüttdichte in kg/L wurde bestimmt mit Hilfe eines 1 L Messzylinders, der mit dem Schüttgut genau bis zum Marke 1000 ml aufgefüllt wurde und per Wägung das Gewicht des Inhalts gemessen wurde, so dass sich damit direkt die Schüttdichte

### Bestimmung des Fließverhaltens

Das Fließverhalten (Rieselfähigkeit) wurde durch Vergabe einer Rieselnote von 1 (sehr gut) bis 6 (ungenügend) bestimmt aufgrund vorheriger Messung der Rieselfähigkeit mit Hilfe von Standardauslaufgefäßen aus Glas. Diese hatten eine zylindrische Form mit konischem unteren Ende, in dessen Mitte eine Auslauföffnung positioniert war (analog wie bei einem Silo) mit unterschiedlicher Weite von eng für Note 1 bis relativ weit für Note 6. Die Messung wurde mit dem engsten Auslaufgefäß begonnen, die Auslauföffnung mit einem Glasplatte zugehalten, das Auslaufgefäß mit dem Schüttgut befüllt, die Glasplatte weggenommen und das Auslaufen beurteilt. Nur wenn ein praktisch ungehemmtes Auslaufen das Schüttgutes beobachtet werden konnte, dann wurde die Note 1 gegeben. Wenn dies nicht der Fall war wurde das nächstweitere Auslaufgefäß verwendet und bei einwandfreiem Auslaufen die Note 2 vergeben, wenn dies wiederum nicht der Fall war wurde das nächst weitere Auslaufgefäß verwendet usw. bis zum weitesten Auslaufgefäß (Gefäß 5). Nur Material, das auch aus diesem Auslaufgefäß nicht ungehemmt ausfließen konnte, wurde mit Note 6 bewertet. Die Standardauslaufgefäße hatten alle eine Höhe des zylindrischen Teils von 70 mm und einen innere Weite von 36 mm. Die Auslaufsöffnungen hatten die folgenden Weiten für Gefäß1: 2,5 mm, Gefäß 2: 5 mm, Gefäß 3: 8 mm, Gefäß 4: 12mm, Gefäß 5: 18 mm.

### Bestimmung des pH-Wertes

Der pH-Wert wurde mit Hilfe einer Glaselektrode in wässriger Lösung und bei einer Temperatur von 20°C gemessen sofern nicht anders angegeben.

Die **Quantitative Bestimmung der Konzentrationen** von Met, Met-Met, Methionyl-DKP usw. erfolgte mit Standard-HPLC Methoden gegenüber einem externen Standard.

### Allgemeine Verfahrensbeschreibung der DKP-Synthese

Eine wässrige Lösung des Dipeptides Met-Met wurde nach Anpassung des pH-Wertes auf 4,5 oder 5 mit Hilfe von entweder Schwefelsäure oder Ätznatron für eine Zeitdauer von 30 - 120 min auf 150 - 180°C aufgeheizt. Nach Abkühlung auf 70°C durch Druckabsenkung wurde eine wässrige Suspension erhalten. Nach Abfiltrieren und Waschen des Filterkuchens mit Wasser wurde ein Feststoff erhalten, der hauptsächlich DKP enthielt, das aus einer Mischung von DLLD-DKP und DDLL-DKP in einem Verhältnis von im günstigen Fall 50/50 oder größer bestand unabhängig von der stereochemischen Konfiguration des Ausgangsmaterials Met-Met.

### Beispiel 1 (Variation des pH - basische Bedingungen, Vergleichsbeispiel)

Durch Anpassung des pH auf 9.3 und ansonsten Durchführung gemäß der allgemeinen Verfahrensbeschreibung wurde hauptsächlich Methionin schon bei Temperaturen von 140°C gebildet. DKP wurde nur in einer Ausbeute von 34% und mit einem Diastereomeren-Verhältnis von 57/43 DLLD-/DDLL-DKP erhalten, ausgehend von einem Diastereomeren-Verhältnis von 36/64 DLLD/DDLL-Met-Met im Ausgangsmaterial.

### Beispiel 2 (Variation des pH - saure Bedingungen, Vergleichsbeispiel)

Durch Anpassung des pH auf 2,9 und ansonsten Durchführung gemäß der allgemeinen Verfahrensbeschreibung wurde hauptsächlich Methionin schon bei Temperaturen von 140°C gebildet. DKP wurde nur in einer Ausbeute von 60% und mit einem Diastereomeren-Verhältnis von 36/64 DLLD-/DDLL-DKP erhalten, ausgehend vom selben Diastereomeren-Verhältnis von 36/64 DLLD-/DDLL-Met-Met im Ausgangsmaterial.

### Beispiele 3 bis 5 (Variation von Temperatur, Reaktionszeit und pH)

### Beispiel 3

Durch Aufheizen der Mischung auf 150°C, halten bei dieser Temperatur für 120 min und anschließend halten bei 120°C und pH 9,0 für 60 min und ansonsten Durchführung gemäß der allgemeinen Verfahrensbeschreibung wurde DKP in 53% Ausbeute erhalten bei deutlicher Epimerisierung. Das Verhältnis der Diastereomere verschob sich von 15/85 DLLD/DDLL-Met-Met auf 60/40 DLLD/DDLL-DKP.

### Beispiel 4

Dagegen bei Aufheizen der Mischung auf 150°C, halten bei dieser Temperatur für 120 min und pH 4,8 sowie ansonsten Durchführung gemäß der allgemeinen Verfahrensbeschreibung wurde DKP in 80% Ausbeute erhalten bei deutlich geringerer Epimerisierung. Das Verhältnis der Diastereomere verschob sich von 15/85 DLLD/DDLL-Met-Met auf 25/75 DLLD/DDLL-DKP.

### Beispiel 5

Hingegen bei Aufheizen der Mischung auf 160°C, halten bei dieser Temperatur für 60 min und ansonsten Durchführung gemäß der allgemeinen Verfahrensbeschreibung wurde DKP in 72% Ausbeute erhalten bei deutlicher Epimerisierung. Das Verhältnis der Diastereomere verschob sich von 15/85 DLLD/DDLL-Met-Met auf 39/61 DLLD-/DDLL-DKP.

### Beispiel 6

Es wurden 1,47 kg einer wässrigen Lösung mit einem Gehalt von 2.9% DDLL-Met-Met (151 mmol) und 0.9% DLLD-Met-Met (50 mmol) durch Zugabe von 10proz. NaOH auf pH 4.5 eingestellt. Anschließend wurde die Lösung schnellstmöglich auf eine Temperatur von 170°C (7barg) aufgeheizt und bei dieser Temperatur für 90 min gehalten. Nachdem kein Met-Met mehr analytisch nachweisbar war, wurde die Reaktionsmischung durch Entspannen auf 100°C abgekühlt. Die dabei erhaltene Suspension wurde unter Vakuum weiter auf 70°C abgekühlt. Nach Abfiltrieren des entstandenen Feststoffniederschlags und Waschen des Filterkuchens mit 150 g Wasser wurden 98 g bräunlicher Kristalle erhalten. Es wurden 43 g einer DKP-Mischung (161 mmol, 80 % Ausbeute) aus beiden Diastereomeren nach Trocknung isoliert in einem Verhältnis von DLLD-/ DDLL-DKP von 48 / 52 mit einer chemischen Reinheit von 98%.

### Beispiele 7 und 8 (Variation der Reaktionszeit)

Durch Verkürzung der Reaktionszeit auf 60 min und ansonsten Durchführung gemäß der Verfahrensbeschreibung unter Beispiel 6 wurde eine niedrigere Ausbeute von 73% DKP mit einem Dia-stereomeren-Verhältnis von 52/48 DLLD/DDLL-DKP erhalten und mit 30 min Reaktionszeit eine Ausbeute von 71% mit einem Diastereomeren-Verhältnis von 48/52.

### Beispiel 9 (Erhöhung der Reaktionstemperatur)

Durch eine weitere Erhöhung der Temperatur auf 175°C, halten für 30 min bei dieser Temperatur und pH 4,5 sowie ansonsten Durchführung gemäß der Verfahrensbeschreibung unter Beispiel 6 wurde wieder eine etwas höhere Ausbeute von 77 % DKP.erzielt im Vergleich zu Beispiel 8 mit einem Diastereomeren-Verhältnis von 38/62 DLLD-/DDLL-DKP.

### Beispiel 10 (Hydrolyse von DLLD/DDLL-Met-DKP zu DLLD/DDLL-Met-Met etc.)

Die gemäß der Beispiele 3 bis 9 erzeugten Diastereomerengemische von DLLD/DDLL-Met-DKP konnten gemäß der Schritte e. bis h. (siehe oben bzw. Fig.1) zu den entsprechenden Gemischen von DLLD/DDLL-Met-Met-Alkalisalzen hydrolysiert werden. Dabei wurde nach entsprechendem Ansäuern mit beispielsweise Schwefelsäure bis auf pH 4,5 bis 5,6 DLLD-Met-Met als Feststoff ausgefällt, abfiltriert gewaschen und getrocknet. Das so erhaltene DLLD-Met-Met zeigte durchweg Rieselnoten von 1 bis 2, Schüttgewichte von > 600 kg/m3, sowie geringe Staubanteile von ca. 10 % < 63 µm. Die gefundenen Mindestzündenergien dieser Produkte lagen bei Raumtemperatur über 10 mJ und selbst bei 100°C noch über 3 mJ.

**Tabelle1: Überblick über die Beispiele**

| Beispiel | Ausgangsmaterial/Art der Umsetzung | Reaktio ns-zeit [min] | Temp. [°C] | pH | Met-Met-Konz. [Gew%] | Ausbe ute [%] | Met-Met-Startverhältnis DLLD / DDLL | DKP-Endverhältnis DLLD / DDLL |
|---|---|---|---|---|---|---|---|---|
| 1 (Vergleich) | Met-Met-Mutterlauge/ Epimerisierung unter basischen Bedingungen | 120 | 140 | 9,3 | 10,5 | 34 | 36/64 | 57/43 |
| 2 (Vergleich) | Met-Met-Mutterlauge/ Epimerisierung unter sauren Bedingungen | 120 | 140 | 2,9 | 8,9 | 60 | 36/64 | 36/64 |
| 3 | Met-Met-Mutterlauge / Zyklsierung und Epimerisierung am IP (Isoelektrischen Punkt) gefolgt von basischen Bedingungen | 120 | 150 | 4,5 | 3,4 | 53 | 15/85 | 60/40 |
| | | 60 | 120 | 9,0 | | | | |
| 4 | Met-Met-Mutterlauge/ Epimerisierung am IP | 120 | 150 | 4,8 | 3,4 | 80 | 15/85 | 25/75 |
| 5 | Met-Met-Mutterlauge/ Epimerisierung am IP | 60 | 160 | 4,5 | 3,4 | 72 | 15/85 | 39/61 |
| 6 | Met-Met-Mutterlauge/ Epimerisierung am IP | 90 | 170 | 4,5 | 3,4 | 81 | 15/85 | 48/52 |
| 7 | Met-Met-Mutterlauge/ Epimerisierung am IP | 60 | 170 | 4,5 | 3,4 | 73 | 15/85 | 52/48 |
| 8 | Met-Met-Mutterlauge/ Epimerisierung am IP | 30 | 170 | 4,5 | 3,4 | 71 | 15/85 | 48/52 |
| 9 | Met-Met-Mutterlauge/ Epimerisierung am IP | 30 | 175 | 4,5 | 3,4 | 77 | 15/85 | 38/62 |

## Patentansprüche

1. Verfahren zur Herstellung von DL/LD-Methionindiketopiperazin (I) 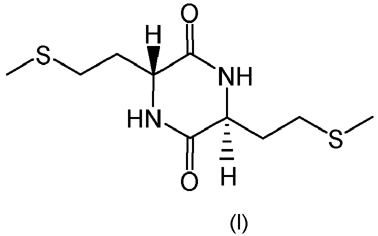 **dadurch gekennzeichnet, dass** man eine DD/LL-Methionyl-methionin enthaltende wässrige Lösung oder Suspension, die einen pH-Wert von 3 bis 9, gemessen mit einer pH-Elektrode bei 50 °C aufweist, solange bei einer Temperatur von 150 bis 220°C, vorzugsweise bei 170 bis 210 °C umsetzt, bis eine wässrige Lösung oder Suspension enthaltend mindestens einen Anteil von 30 Mol %, vorzugsweise 50 Mol %, insbesondere 60 Mol% DL/LD-Methionindiketopiperazin bezogen auf den Gesamtgehalt an DL/LD- und DD/LL-Methionindiketopiperazin im Reaktionsgemisch entstanden ist.

2. Verfahren gemäß Anspruch 1, **dadurch gekennzeichnet dass** die Umsetzung bei einem pH-Wert von 4 bis 6 erfolgt.

3. Verfahren gemäß Anspruch 1 oder 2, **dadurch gekennzeichnet dass** man bei einer Temperatur von 175 bis 210 °C umsetzt.

4. Verfahren gemäß einem der Ansprüche 1 bis 3, **dadurch gekennzeichnet dass** die Gesamtkonzentration an DL/LD- und DD/LL-Methionyl-methioninäquivalenten in Form von DL/LD- und DD/LL- Methionindiketopiperazin im Reaktionsgemisch am Ende der Reaktion im Bereich von 0,1 bis 18 Gew.%, vorzugsweise im Bereich von 1 bis 15 Gew.%, liegt.

5. Verfahren gemäß einem der Ansprüche 1 bis 4, **dadurch gekennzeichnet dass** man eine DD/LL-Methionyl-methionin enthaltende wässrige Lösung oder Suspension, die bereits DL/LD-Methionyl-methionin enthält einsetzt.

6. Verfahren gemäß Anspruch 5, **dadurch gekennzeichnet dass** die eingesetzte wässrige Lösung oder Suspension bereits bis zu einem Anteil von 30 Mol% DL/LD-Methionylmethionin enthält bezogen auf ihren Gesamtgehalt an DD/LL- und DL/LD - Methionyl-methionin.

7. Verfahren zur Gewinnung von diastereomerenreinem DL/LD-Methionylmethionin, **dadurch gekennzeichnet, dass** man
a. Methionin-Hydantoin zusammen mit Alkalibase umsetzt zu einem Diastereomerengemisch aus DL/LD-Methionindiketopiperazin und DD/LL-Methionindiketopiperazin (DLLD-DKP und DDLL-DKP)
b. das DLLD-DKP und DDLL-DKP aus a. durch Einengen oder Abkühlen der Reaktionslösung aus a. zur Kristallisation bringt
c. das auskristallisierte DLLD-DKP und DDLL-DKP abtrennt von der DKP-Mutterlauge
d. das abgetrennte DLLD-DKP und DDLL-DKP in Wasser einmischt und das Gemisch durch Zugabe entsprechender Mengen Alkali auf einen pH-Wert von 10 bis 14, vorzugsweise 13, gemessen mit einer pH-Elektrode bei 20 °C, bringt und bei einer Temperatur von 90 bis 150 °C und einer Verweilzeit von 30 bis 180 min zu einer entsprechenden wässrigen Lösung oder Suspension eines Diastereomerengemisches aus DL/LD-Methionylmethionin- und DD/LL-Methionylmethionin-Alkalisalz hydrolysiert,
e. den pH-Wert der Lösung oder Suspension aus d., gemessen bei 20°C, durch Zugabe entsprechender Mengen Säure auf < 9,0, vorzugsweise auf 4,6 bis 5,6, besonders bevorzugt auf 5,0 bis 5,2 absenkt
f. durch Verdampfen von Wasser und/ oder Abkühlen eine Suspension erzeugt, die aus einem Feststoffanteil, der vorwiegend DL/LD-Methionylmethionin enthält, und Mutterlauge, die vorwiegend DD/LL-Methionylmethionin enthält, besteht, man
g. den Feststoffanteil aus f. von der Mutterlauge abtrennt, und man
h. ihn wahlweise wäscht und/oder trocknet, und dabei diastereomerenreines DLLD-Methionylmethionin erhält und man
i. die verbleibende DD/LL-Methionylmethionin enthaltende Mutterlauge aus g. gemäß Anspruch 1 umsetzt, so dass eine wässrige Lösung oder Suspension enthaltend ein Diastereomerengemisch aus DL/LD-Methionindiketopiperazin und DD/LL-Methionindiketopiperazin entsteht mit einem Anteil von bis zu 60 Mol% DL/LD-Methionindiketopiperazin am Gesamtgehalt der DL/LD- und DD/LL-Methionylmethioninäquivalente im Reaktionsgemisch und
j. diese dann in Schritt b. zurückführt

8. Verfahren gemäß Anspruch 7, **dadurch gekennzeichnet, dass** man in Schritt d. 0,9 bis 1,5 Moleq, vorzugsweise 1,0 bis 1,2 Moleq Base bezogen auf den Gesamtgehalt an DL/LD- und DD/LL-Methionyl-methioninäquivalenten im Reaktionsgemisch einsetzt.

9. Verfahren gemäß Anspruch 7 oder 8, **dadurch gekennzeichnet, dass** man in Schritt d. Alkalimetallhydroxide, vorzugsweise NaOH oder KOH als Base verwendet.

10. Verfahren gemäß einem der Ansprüche 7 bis 9, **dadurch gekennzeichnet, dass** man in Schritt e. starke Mineralsäuren, vorzugsweise Phosphorsäure, Salzsäure oder Schwefelsäure, insbesondere Schwefelsäure verwendet.

11. Verfahren gemäß einem der Ansprüche 7 bis 10, **dadurch gekennzeichnet, dass** man in Schritt f. bis zu einer Temperatur von ≥ 50 °C abkühlt.

12. Verfahren gemäß einem der Ansprüche 7 bis 11, **dadurch gekennzeichnet, dass** die in Schritt f. erzeugte Suspension mindestens 1 Gew%, jedoch nicht mehr als 15 Gew.% Gesamtgehalt an DD/LL- und DL/LD -Methioninyl-methionin enthält.

13. Pulverförmiges diastereomerenreines DL/LD-Methionylmethionin, erhältlich nach einem Verfahren gemäß einem der Ansprüche 7 bis 12 zur Verwendung als Futtermittelzusatzstoff für Aquakulturen.

14. Pulverförmiges diastereomerenreines DL/LD-Methionylmethionin enthaltendes Produkt, welches die folgenden Merkmale aufweist:
a. Mindestens 97 Gew.% Gesamtgehalt an DD/LL- und DL/LD -Methionyl-methionin,
b. Mindestens 90 Mol% DL/LD-Methionylmethionin und maximal 10 Mol% DD/LL-Methionyl-methionin, vorzugsweise mindestens 95 Mol% an DL/LD-Methionyl-methionin und maximal 5 Mol% DD/LL-Methionyl-methionin, jeweils bezogen auf den Gesamtgehalt an DD/LL- und DL/LD -Methionyl-methionin,
c. Max. 1,0 Gew.% Restfeuchte,
d. Max. 2,0 Gew.% Sulfat(Asche) und
e. einer Korngrößenverteilung mit einem Median D50 von 80 bis 300 µm, vorzugsweise 150 bis 250 µm
zur Verwendung als Futtermittelzusatzstoff für Aquakulturen.

15. Verwendung von diastereomerenreinem DL/LD-Methionyl-methionin gemäß Anspruch 14 als Futtermittelzusatzstoff für Aquakulturen.
